# EUROPEAN PATENT SPECIFICATION

(11) **EP 4 389 037 B1**
(45) Date of publication and mention of the grant of the patent: **17.09.2025**
(21) Application number: 23210439.8
(22) Date of filing: 16.11.2023
(51) Int. Cl.: A61B 18/12, A61B 17/00, G06F 11/30

(54) **ELECTRO SURGICAL GENERATOR AND METHOD FOR OPERATING AN ELECTRO SURGICAL GENERATOR**
ELEKTROCHIRURGISCHER GENERATOR UND VERFAHREN ZUM BETREIBEN EINES ELEKTROCHIRURGISCHEN GENERATORS
GÉNÉRATEUR ÉLECTROCHIRURGICAL ET PROCÉDÉ DE FONCTIONNEMENT D'UN GÉNÉRATEUR ÉLECTROCHIRURGICAL

(30) Priority: 23.12.2022 US 202263435081 P
(43) Date of publication of application: 26.06.2024
(73) Proprietor: Olympus Winter & Ibe GmbH, 22045 Hamburg (DE)
(72) Inventor: Krüger, Jens, 15738 Zeuthen (DE); Stopp, Fabian, 10318 Berlin (DE); Kwik, Anne, 39590 Tangermünde (DE)
(74) Representative: Eisenführ Speiser

(56) References cited:
- US-A1- 2014 253 140
- US-A1- 2018 210 801
- US-A1- 2020 078 077
- US-B2- 6 830 569

## Description

The present invention is directed to a method for operating an electro surgical generator.

The invention is also directed to such electro surgical generator. A system supplying a test signal and configured to detect an abnormality within a circuit of an electrosurgical generator is known from US 2014/253140 A1.

Electro surgical generators control electro surgical instruments connected to such generator. In particular there is at least one inverter for generating a high frequency feed signal in order to feed an electro surgical instrument connected to the electro surgical generator. For safety applications in such generators the generators should ensure that components are able to react on an input, such as measurements, with a correct output, in particular the control of the feed signal, i.e. the control of provided high frequency energy, in a timely manner. To enable this, components such as an inverter module can be tested for reactivity. If a component is stuck or has a malfunction, this can be detected via a frequent test routine. In such frequent test routine for each relevant component it is checked whether it is still operating properly, at least if it is still sending confirmation signals on a frequently basis. Such frequent test can be called a heartbeat system as it frequently checks whether the particular component is still in operation.

Such reaction time on failures can be defined as failure tolerance time (FTT).

In a worst case scenario the generator outputs a maximum HF energy power which is not controllable. In the tissue of the patient this energy generates heat and burns the tissue, resulting in carbonization. The failure tolerance time takes this failure scenario and calculates a time after which the tissue is damaged irreversibly. In a failure scenario the safety system must be able to shut down the generator in this timeframe. As an example for a generator which can output 2500 W in a single fault condition, the failure tolerance time can be calculated to be about 200 ms or in one example 189 ms.

In a heartbeat system the supervision time should be well below the failure tolerance time to ensure that a malfunctioning subcomponent is detected and there is enough time left to shut down the HF energy output. However, this creates a high amount of communication traffic because every node which can be representative of a component or module connected to a communication network, has to send data at least one or more times in this timeframe. Accordingly, each connected component, in particular module sends data at least every 200 ms according to the example given above. That is a minimum and if it sends two times in said timeframe, it sends data every 100 ms. Not only one module but all modules connected to said network may thus send within that timeframe, resulting in a lot of communication traffic.

In a distributed communication system, such as a system with nodes connected to a CAN, this can lead to high bus load for this communication.

According to one prior art a control software of a generator may be distributed on various modules. A central module may send a signal, which can be called a heartbeat signal, to all other modules which need to answer within a predetermined time. Otherwise, the generator changes over into an error state.

Accordingly, the object of the present invention is to provide a solution to at least one of the above explained problems. In particular, a solution shall be suggested that entrusts a high safety level but at the same time avoiding an overload of a communication system in particular a communication network. At least an alternative solution with respect to known solutions shall be suggested.

According to the invention a method according to claim 1 is suggested. Accordingly a method for operating an electro surgical generator is suggested. Such electro surgical generator is adapted for controlling electro surgical instruments connected to the electro surgical generator. In particular the generator provides HF energy to at least one electro surgical instrument. Further control tasks are executed by such generator such as receiving and forwarding inputs from a user and displaying states of the generator.

The electro surgical generator used comprises a plurality of interconnected regular modules comprising at least one socket module for connecting an electro surgical instrument. Such socket module may also be denominated as output module. The electro surgical instrument may be plugged into such socket module. The socket module may comprise a sensor indicating whether and possibly what kind of electro surgical instrument is connected. The socket module may also have a sensor checking an amplitude of a feed signal. The socket module may also have an information connection receiving information on an operating state of the connected electro surgical instrument. Any such information received can be communicated inside the electro surgical generator to other modules in the generator and/or to a communication module. There may be a communication between the socket module and an inverter module which provides the feed signal to the socket module and thus to the connected electro surgical instrument.

The regular modules also comprise at least one first inverter module for generating a feed signal for providing a high frequency energy for at least one electro surgical instrument connected to the socket module. Such inverter module may thus generate, by using a pulse width modulation, a voltage signal having a particular frequency and resulting in a corresponding current signal delivered to the socket module and thus to a connected electro surgical instrument. The high frequency energy is thus energy provided by a feed signal having a frequency in the range of 20 kHz to 500 kHz.

It is further suggested that each regular module communicates at least with another regular module or with a communication module. Accordingly, all these regular modules are at least adapted for communication. The regular modules may communicate directly with each other and/or via the communication module. Accordingly, a centralized as well as a decentralized communication, i.e. network architecture can be possible. It is suggested that an inverter module communicates with a socket module. The socket module may receive from a lever or switch from the connected electro surgical instrument a request for providing a feed energy or a request for stopping to supply the feed energy. Accordingly, this information is communicated with the corresponding inverter module. The inverter module is accordingly generating the wanted high frequency feed signal. In the same manner a request for stopping the feeding can be received by the socket module and communicated to the inverter module.

It is further suggested that each regular module communicates at least with another regular module and/or with a communication module. For this purpose of communication each of the regular modules and/or communication module may have a node for participating in the communication and in particular for participating in a network system such as a network bus system. By means of this communication the operation of the regular modules can be controlled. For this purpose each of the regular modules may have its own control module and accordingly each regular module may be provided as an intelligent module that can receive information, send information and control its own operation based on such information.

It is further suggested that each regular module is sending a communication frame at an individual repetition frequency. Accordingly, one regular module can send a communication frame every 100 ms, whereas another regular module may send such communication frame every second. And accordingly, it is suggested that the individual repetition frequency of each regular module is different to the repetition frequency of at least one, a plurality or all of the other regular modules.

Accordingly, all regular modules may have different repetition frequencies so that in case of 5 regular modules there are 5 repetition frequencies. However, it is also possible that similar regular modules and/or regular modules that are in similar operation mode have the same or similar repetition frequency. The repetition frequency can also be denominated as heartbeat frequency or heartbeat frame frequency.

Accordingly, with respect to a known system explained above the generator is modified such that the high failure tolerance time related heartbeat frame frequency can be altered with a lower frequency if the generator is not in an activation state. It was found that failures that occur without contact to the patient and without any HF output have nearly no risk involved. In these cases, a stuck subcomponent, i.e. a stuck regular module may lead to a lower reaction time for example when a user interface freezes, or a delayed activation. Accordingly, regular modules that are not having such safety relevance may have a lower repetition frequency than regular modules which do have such high safety relevance. In particular an inverter module currently generating a high frequency feed signal thus providing high frequency energy to an electro surgical instrument shall have a high repetition frequency. Such inverter module must quickly stop its operation in case of a malfunction or other error occurring. Such inverter module may provide harmful energy to the patient and accordingly must be stopped quickly in case of any problems occurring.

The output module, to which the electro surgical instrument is connected, may also use a high repetition frequency, such as 10 or 20 Hz. Such socket module may receive any information of a malfunction or of a stop signal of the connected elector surgical instrument. Such signal must be forwarded quickly to the corresponding inverter module. Accordingly, the socket module may also provide a safety relevant function.

It is therefore also suggested to check the correct operational functioning of such socket module and/or of such inverter module. This can also be done by evaluating whether each of these modules send a data frame regularly, i.e. with the repetition frequency. Accordingly, if the repetition frequency of such module is 10 Hz, it is checked each 100 ms whether the particular module is still in operation.

On the other hand the display module which is also a regular module, can use a repetition frequency of only 1 Hz or maybe even a lower repetition frequency. Displaying any information with a delay of one second is not a big problem. Also identifying a malfunction of the display module after one second should also not be a problem as this will not lead to hurting the patient. That will also not lead to any other significant problem for the patient or the electro surgical generator or the attached electro surgical instrument.

However, it could also be possible that the repetition frequency of each or at least some modules changes depending on their operational state. If the inverter module is in operation a higher repetition frequency is advisable, as explained above. However, if the inverter module is not in operation and in particular if an electro surgical instrument is not even connected, a lower repetition frequency may then be sufficient.

At the same time there could be another inverter module active feeding another electro surgical instrument connected, if the electro surgical generator comprises more than one inverter module and more than one socket module.

Accordingly, the communication or bus load during standby of the device is reduced. There is thus the advantage of a lower communication load. There is also the advantage that during standby the generator is ready for a user input, like selecting modes and configuring settings, loading pictures or saving logs. This can be much smoother if there is a reduced intertask communication or reduced heartbeat frequency. It was thus found that in case of a high repetition frequency of all modules participating in the communication, the corresponding network might be overloaded such that the operation of some functions may be delayed. To give an example if the user configures settings, the user will thus use a corresponding interface, possibly an interface having a touchscreen. If the user tries to change a setting, such change of setting is communicated from the interface module to a communication module and possibly further to other modules, as the inputs given by the user might need to be confirmed and/or when changing to a submenu on the touchscreen the submenu might at the first **place** need to be loaded from the communication module or from another module in the generator. This needs communication capacity of the communication network and if this communication network is overloaded, such communication may be delayed and this can be recognized by the user as delays in loading the submenu. However, that is just an example and the overall impression of the user might be, that the system is not working smoothly.

According to one aspect the electro surgical generator further comprises the communication module for controlling a communication with the regular modules and/or each regular module frequently sends a communication frame at its individual repetition frequency to the communication module and/or to another regular module. As the communication module may have a supervisory function it can also be denominated as supervisory communication module.

Accordingly, the communication module is used for controlling the communication within the electro surgical generator. This way a centralized controller of the communication can be established and the communication module can act as one supervising node whereas one or more regular modules are connected as regular nodes to the supervising node. The connection point or connection interface of the communication module to the communication network in particular to the bus system can thus be denominated as the supervising node. Similar the connecting point or interface of a regular module to the communication network in particular to the bus system can be denominated as a regular node.

Preferably each node or module can send a CAN-frame which has at least two information, namely the information of the own node ID of the corresponding node and as a second information an inner state of operation. Such inner state of operation can for example be a boot-up, an error information, the information that the module is in standby, or the information that the module is busy, to give just some examples.

It is possible that additional information like error codes or detailed error information can be part of the CAN-frame and will thus be sent by each node.

Also during operation of the generator, each node regularly sends a heartbeat frame to the supervising node. Such heartbeat frame is thus a frame that is sent frequently with the repetition frequency. The frame sent may just comprise an information indicating that the particular module sending the frame is not having an error. The frequency in which these frames are sent, depends on the current state of the generator, in particular if the generator as such is in standby or in operation or in another mode, or it may depend on the state of the particular modules according to the possible inner states of operation mentioned above.

This way a high repetition frequency is possible for any safety relevant operation of the generator or of particular modules. For all other modules and/or situations, a lower repetition frequency is used and thus that enables to reduce a load on the communication system, in particular on the bus system.

According to one aspect it is suggested that the repetition frequency of at least one of the regular modules is given by the communication module, wherein the communication module sends an individual repetition frequency setting to the regular module and the regular module records the received repetition frequency setting.

Alternatively the communication module controls the repetition frequency of a regular module by sending at the repetition frequency communication frames to the regular module requesting an instantaneous answer and thereby forcing the regular module to answer at the repetition frequency.

According to both ways the communication module controls the particular repetition frequencies of the modules and thus can control the overall operation and can thus also control the load on the communication network.

When sending said repetition frequency setting that could be by sending a corresponding frame comprising the repetition frequency. In that case the frame might in addition comprise the node ID of the corresponding regular module and the repetition frequency that shall be used. The regular module receives this data frame and stores the repetition frequency provided that way. Accordingly, the particular regular module sends at least heartbeat frames with this repetition frequency just stored. That can be done until the communication module sends a new individual repetition frequency setting to that regular module. This new repetition frequency is then stored instead of the previously stored repetition frequency and accordingly the regular module sends with this new repetition frequency.

If the communication module sends at the particular repetition frequency of a particular regular module communication frames to this particular module, the module has to react and respond almost instantaneously. This way the regular module also sends at the repetition frequency provided by the communication module, but without recording such repetition frequency. This repetition frequency may also easily change whenever the communication module sends said communication frames with a new repetition frequency.

In both cases the communication traffic between the communication module and the particular regular module can be controlled. As the communication module may control all regular modules in this way, the communication module also controls the overall information traffic on the communication system, in particular on the bus system.

Accordingly, the communication module and thus the communication node controls the heartbeat rate of all other CAN nodes, if a CAN-bus system with CAN-nodes is used, i.e. it controls the heartbeat rate of the regular modules. Accordingly, the one possibility is given, namely the communication module or communication node sends the frequency setting to the node of a regular module and that node saves this setting until the supervising node is sending a new change, i.e. a new frequency setting.

According to the other possibility, the communication module sends a frame that must be answered immediately, at least within a given acceptable time frame which can be in the range of 5-100 ms, preferably 5-20 ms. This frame must be answered immediately from the node and this way the communication module controls directly the rate of the heartbeat at each node.

Setting the repetition frequency of the regular modules by the communication module can have the following advantage. A module specific handling and specific risks can be achieved. For example a temperature sensor does not need to be supervised very fast because failures would not immediately result in critical situations. Another advantage is the modular approach. Independent development of each modules with their own specification is possible. This also opens or facilitates the possibility of exchanging such modules, as these modules have individualized repetition frequencies and thus exchanging a module leads to taking another module which can also have its own repetition frequency.

Another advantage is that such solution is easy to implement. One possibility to implement is that any new or exchanged regular module can just receive its repetition frequency by the communication module and thus this can be controlled by the communication module in a centralized way.

It was however also realized that information about a particular heartbeat frequency, i.e. the particular repetition frequency, can be programmed in the communication module or communication node and thus in case of changes of a module, if for example a new module is mounted, that may require a change of the communication module or at least the communication node. However, such change can be preprogrammed and stored in a corresponding configuration file stored in the communication module.

According to one aspect at least one of the regular modules, in particular a plurality of the regular modules or all of the regular modules has its individual repetition frequency recorded in a memory as an individual repetition frequency setting, and provides its individual repetition frequency to the communication module and/or provides its individual repetition frequency to other regular modules, whereby the regular modules or the communication module check whether the sent individual repetition frequency complies with a check routine.

According to this aspect each regular module has its own repetition frequency stored. The communication module and/or at least one other regular module can check whether said regular module sends at its repetition frequency. To be able to check that, the repetition frequency of each regular module is thus sent to the other modules, i.e. to the communication module and/or one or more other regular modules. The underlying idea is that, in particular, the type of regular module is closely connected to its repetition frequency. Accordingly, it is possible to configure each regular module with its repetition frequency right from the start. However, it is also possible to change the repetition frequency. And changing the repetition frequency may be executed by each regular module itself which is however then reported to the other regular modules and/or the communication module.

According to one aspect the individual repetition frequency is used to check a reactivity of the corresponding regular module, wherein for checking the reactivity, a test signal is sent to the regular module to be tested, and it is monitored if the regular module answers the test signal at least at its repetition frequency.

Accordingly, it can be ensured for each regular module whether it is free of any problems. That is done by checking, if each regular module reacts on a corresponding test signal. Such test signal can be a data frame sent to each regular module including an order to send a response. Such response is sent within the repetition frequency of the particular regular module, if said regular module is operating correctly. Accordingly, if the particular repetition frequency is 10 Hz, then the response to such test signal shall be within 100 ms. It might also be possible that said test signal and in particular said data frame sent comprises an order to frequently send a response. In other words it might be possible to send one test signal and receive a plurality of responses, whereas the plurality of responses is sent with the individual repetition frequency.

This way the functioning of each regular module can be ensured which is quite important for an electro surgical generator as such generator has a high safety requirement, as any malfunction of a module and correspondingly a malfunction of the electro surgical generator may cause a significant injury of the patient.

According to one aspect individual repetition frequencies of the regular modules are varied, in particular individual repetition frequencies are varied by the communication module and/or individual repetition frequencies are varied depending on operational conditions of the electro surgical generator and/or the corresponding regular module. As explained above, the individual repetition frequency can thus be adapted to particular situations. The individual repetition frequency can be low if the particular regular module is in a standby operation or if the whole electro surgical generator is in a standby situation. If the regular module is in operation the individual repetition frequency can be selected to be higher as compared to any standby situation.

The variation can be done by the communication module, as also outlined above. This way the individual repetition frequencies are controlled in a central manner and this way the repetition frequencies of all modules of the electro surgical generator can be controlled in a central way and adapted to each other.

In addition or alternatively the individual repetition frequencies are varied depending on operational conditions either of the electro surgical generator as a whole or depending on operational conditions of the corresponding regular module. This can nevertheless be performed centrally by the communication module.

According to one aspect the individual repetition frequency is selected depending on the kind of regular module, in particular whether the regular module is involved in controlling energy of an electro surgical instrument connected to the electro surgical generator and/or depending on an operating condition of the regular module, in particular whether the regular module is in operation or in standby.

The kind of regular module may specify which particular regular module is used, i.e. whether it is an inverter module, a socket module, an input interface module or a display module, to give just some examples. As explained above a display module may have a smaller repetition frequency than an inverter module, as it is expected that the display module is less safety relevant than the inverter module.

Selecting the repetition frequency depending on an operational condition of the regular module has already been explained above. In particular, when the regular module is in operation, a higher repetition frequency is used, whereas a smaller repetition frequency is used if the regular module is in standby.

According to one aspect the plurality of interconnected regular modules comprises at least one further module as follows. One regular module may be an energy distribution module for controlling an energy supply from the at least one inverter module to the at least one socket module. For such energy distribution module the energy supplied from the at least one inverter module to the at least one socket module can pass through the distribution module and can this way be controlled. However, it is also possible that the energy, i.e. the feed signal generated by the inverter module, is directly supplied to the socket module, i.e. by a wire directly connecting said inverter module and said socket module. However, also in this case the distribution module may control the particular inverter module and/or it can control any switches provided on a path between an inverter module and a socket module.

The distribution module can be combined with the communication module. In that case part of this combined module in particular the part of physically controlling the energy distribution can form a regular module, whereas the communication module can be understood as a submodule attached to the distribution module.

Another regular module may be a display module for displaying information for a user. Such display module may be connected to the communication module and receiving any information that needs to be displayed from that communication module. Any information to be displayed may be, if the electro surgical generator is busy or in standby. Another information may be a configuration of the electro surgical generator, which has been inputted by the user beforehand. The display module may as well display a temperature of the generator in particular of a regular module of the generator. Another information to be displayed may be a status of connected electro surgical instruments.

Obviously, all these information can be related to different modules and thus all these information can be collected by the communication module and provided by the communication module to the display module. However, in a decentralized structure the information may as well be directly sent or otherwise provided by the corresponding regular modules and also the communication module to the display module.

Another regular module may be an input module for receiving demands from the user. The input module may also be denoted as input interface. Such input module may comprise switches or a touch screen for the user to input demands or other information. An input module or input interface module may also comprise an on/off switch. Such on/off switch could be an own physical device or it can be part of a touch screen.

Another regular module may also be at least a second inverter module for generating a feed signal for providing a high frequency energy for at least one electro surgical instrument with a different signal frequency compared to the first inverter module. In particular, one of the first and second inverter modules may be an inverter module for generating an ultrasonic frequency signal for feeding a corresponding ultrasonic electro surgical instrument. The other inverter module may generate a feed signal of even higher frequency. Even though such inverter may vary its output frequency, the task of generating a feed signal for controlling a supersonic electro surgical instrument is much different to generating a feed signal of a higher frequency which is used to provide an electric current for cutting and/or burning tissue in the body of the patient.

All these regular modules may be interconnected for communication directly and/or via the communication module.

According to one aspect the electro surgical generator comprises a monitoring module for monitoring if the communication module operates properly. In particular the communication module may monitor if all regular modules operate properly. That is particularly done by checking if each regular module responses with its individual repetition frequency. However, it is also important that the communication module works properly and for doing this it is suggested to use such monitoring module. This monitoring module may have the only purpose to monitor the communication module. The communication module in turn may monitor whether the monitoring module works properly. This way it can of course hardly be avoided that both modules do not work properly but at least a redundancy can be provided by using this monitoring module.

According to one aspect the regular modules and the communication module are interconnected using a bus system, in particular a serial and/or differential bus system. In particular, a CAN-bus is used, which is a serial bus system. It was found that such bus system, in particular, the serial bus system and CAN-bus system provides the possibility to interconnect a plurality of nodes, i.e. a plurality of regular modules within adequate transmission rate but at the same time being resistant against noise. This is important as the used inverter module or inverter modules may produce such noise due to their operation of generating high frequency signals.

According to one aspect each regular module sends a frame comprising at least an identification code identifying the operational mode of the regular module, and an operation code, characterizing the operating state of the regular module, and optionally additional information including information of boot-up, error information, information on standby and information on a busy-status.

Such frame can be used to exchange information with other regular modules and/or with the communication module. Such frame may also be used to send or receive the particular repetition frequency.

According to one aspect an error signal is generated, if a regular module is in a mode in which it should frequently be sending a communication frame at its individual repetition frequency, but the regular module is deviating from sending a communication frame at its individual repetition frequency by a predetermined minimum tolerance frequency. Such regular mode could be a test mode or a reactivity test mode in which the communication is focused on testing whether the particular regular module is without any error or malfunction.

To check this there can be a predetermined minimum tolerance frequency, which can be in a range of 10% to 50% of the repetition frequency. If the regular module is deviating from sending a communication frame at this repetition frequency, an error is indicated. Such error is indicated if the repetition frequency deviates by the predetermined minimum tolerance frequency. However, often such deviation from the individual repetition frequency maybe not sending any response at all. However, in order to better program such check algorithm and in order to immediately identify such malfunction said minimum tolerance frequency can be used.

In addition or alternatively for checking the reactivity a test signal is sent to the regular module to be tested and an error is assumed if an answer of the regular module to the test signal takes longer than a response time. The response time may be defined by an inverse value of the repetition frequency of the tested regular module. The error is assumed if the answer takes longer than the response time by a minimum time lag. So in that case an error signal is also generated. This kind of testing is similar to the first possibility but it is directly comparing a time lag of a response and the minimum time lag considered may be the inverse value of the predetermined minimum tolerance frequency. The minimum time lag may be in the range of 10 to 30% of the inverse of the repetition frequency.

According to the invention there is also suggested an electro surgical generator for controlling electro surgical instruments connected to the electro surgical generator and the electro surgical generator comprises
- a plurality of interconnected regular modules, the plurality of interconnected regular modules comprising
- at least one socket module for connecting an electro surgical instrument, and
- at least one first inverter module for generating a feed signal for providing a high frequency energy as a feed signal for at least one electro surgical instrument connected to the socket module,
wherein
- each regular module is adapted for communicating at least with a communication module,
- each regular module is adapted to send a communication frame at an individual repetition frequency and
- the individual repetition frequency is varied depending on a state of the electro surgical generator.

Preferably the electro surgical generator is adapted to execute a method according to any of the aspects mentioned above.

Accordingly, the electro surgical generator operates as explained above with respect to any aspects of the method for controlling such electro surgical generator. For executing the method by the electro surgical generator such method can be implemented on a control module of the electro surgical generator. Such control module may be the communication module. However, as many steps are executed on different modules in the electro surgical generator the corresponding steps can be implemented at the respective modules. Accordingly, each regular module of the electro surgical generator may have a control module, which could also be a software or a processor connected to the regular module, or a software implemented on such processor. The particular method steps can be implemented on said respective regular module or its control module or on the communication module.

According to one aspect the electro surgical generator further comprises the communication module for controlling a communication with the regular modules, and/or each regular module is adapted to frequently send a communication frame at its individual repetition frequency to the communication module and/or to another regular module.

Accordingly, the electro surgical generator comprises such communication module which can be used as a central element for controlling the communication with the regular modules, as explained above. Each regular module may have stored its individual repetition frequency and is this way adapted to frequently send a communication frame at its individual repetition frequency. Besides that such regular module is in particular adapted to communicate using a bus system and via this bus system the communication frame can be send at its individual repetition frequency.

According to one aspect the plurality of interconnected regular modules comprises at least one further module of the list defined by
- an energy distribution module for controlling an energy supply from the at least one inverter module to the at least one socket module,
- a display module for displaying information for a user,
- an input module for receiving demands from the user, and
- at least a second inverter module for generating a feed signal for providing a high frequency energy for at least one electro surgical instrument with a different signal frequency than the first inverter module.

In addition or alternatively, the electro surgical generator comprises a monitoring module for monitoring if the communication module operates properly.

Accordingly, all these regular modules mentioned can have their own repetition frequency but some may also have a similar repetition frequency. In particular, the display module and the input module may have a much smaller repetition frequency, in particular at least 5 to 10 times smaller than the repetition frequency of the first inverter module, the second inverter module and/or the socket module. The energy distribution module may also have a high repetition frequency similar to the repetition frequency of the first and/or second inverter module and/or the socket module.

The monitoring module may have a high repetition frequency, in particular as high as the first inverter module, the second inverter module and/or the socket module

According to one aspect it is suggested that the regular modules and the communication module are interconnected using a bus system, in particular a serial and/or differential bus system. Accordingly, a communication network inside the electro surgical generator is built up using such bus system, in particular a CAN bus system. This makes the communication resistant to electro smog.

According to the invention it was realized that a constant heartbeat frequency is inflexible in view of the different requirements of the modules in particular of the regular modules. Accordingly, the following solution, also explained above, was found.

Each regular module sends an own heartbeat having an own repetition frequency. The repetition frequency of the modules can be different to each other. The repetition frequencies maybe submitted at a start-up phase by the communication module, which can also be denoted as a central module, for each single regular module. Alternatively, each regular module has its own repetition frequency and communicates this to the communication module, i.e. to the central module.

Such communication module thus supervises the other regular modules, if the heartbeat frequency, i.e. the individual repetition frequency corresponds to predetermined values. A second module, i.e. the monitoring module monitors the central module. The central module may advise other regular modules to send with the higher or lower frequency the heartbeat.

The invention will now be explained by way of example based on the attached Figures.
- Figure 1: shows an electro surgical generator in a schematic view.
- Figure 2: illustrates one control concept of handling the repetition frequency.
- Figure 3: illustrates another control concept on how to handle the repetition frequency.
- Figure 4: illustrates a further control concept on how to handle the repetition frequency.
- Figure 5: illustrates a check routine and load situation of the electro surgical generator using a communication bus.

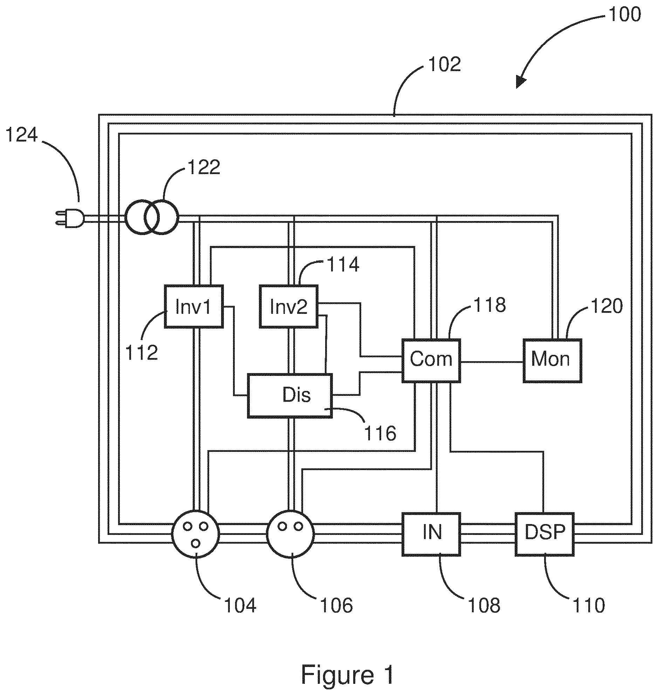

Figure 1 shows in a schematic illustrative way an electro surgical generator 100 having a casing 102 illustrated by a triple line. In the casing 102 there may be mounted a first and second socket module 104 and 106 as well as an input module 108 and a display module 110. So these four modules could be attached at a front plate of the electro surgical generator 100 in the casing 102.

These four modules are regular modules. There are further regular modules, i.e. a first inverter module 112 and a second inverter module 114 as well as a distribution module 116. In addition, there is a communication module 118 and a monitoring module 120.

In addition, there is a power supply 122 illustrated as a transformer and having an electric plug 124.

For illustrative reasons double lines connecting modules or other components are used as a symbol for electric lines transmitting energy, whereas single lines are informational lines and can thus build communication network, in particular a bus system.

Accordingly, the power supply 122 provides electrical energy directly or indirectly to all modules. The input module 108 and the display module 110 may also get electric energy which is just not illustrated in order to not make the Figure to complex.

The first inverter module 112 may be an inverter module providing supersonic feed signal to a corresponding electro surgical instrument that can be plugged into the first socket module 104. The first inverter module 112 thus directly provides the first socket module 104 with energy, if in operation.

The second inverter module 114 may provide an electrical high frequency feed signal which is controlled and guided via the distribution module 116 to the second socket module 106. A different electro surgical instrument can be plugged into the second socket module 106.

For communication the communication module 118 is controlling each of the regular modules, i.e. it is controlling the first and second socket module 104 and 106, the input module 108, the display module 110, the first and second inverter module 112 and 114 and the distribution module 116. This is indicated by a single line connecting each single regular module with the communication module 118. Via these single lines which basically build the communication network and can be a serial bus system or part of it, such as a CAN bus. In addition, there is also a communication connection between the distribution module 116 and the first and second inverter 112 and 114. However, this communication line might not be necessary and any communication between these modules could also go via the communication module 118.

Accordingly, all of these regular modules have a kind of intelligence, i.e. a control module which can be implemented on a processor on each of these regular modules. In this way all of these regular modules are also capable of sending signals indicating their status, in particular if there is an error or not and also if they are in operation or in standby.

For indicating that a regular module is not having an error, there is a heartbeat signal sent from each regular module to the communication module 118. Such heartbeat signal can be a data frame comprising a node ID, namely an identification code identifying the particular regular module and a module state. The module state might just comprise an okay or a not okay. It could also have an information on whether the particular regular module is in standby or in operation and possibly in case there is an error, such error code could also be provided.

However, such data frame is sent with a repetition frequency from each regular module to the communication module 118. Each regular module may have an individual repetition frequency and thus with respect to another regular module a different repetition frequency. In particular, the repetition frequency can be 2 Hz or 12 Hz. Of course, other values could be used as well and possibly there could also be more than two different repetition frequencies.

In addition, there is the monitoring module 120, which is just monitoring the communication module 118. The communication module 118 checks for all the regular modules whether they are operating properly or are not having an error, i.e. if they send a so-called heartbeat signal with the repetition frequency and of course providing an information that everything is okay. This way, all regular modules are checked whether they were properly.

Only the communication module as does not check itself, as the electro surgical generator 100 has to fulfil a high safety requirement. Accordingly, such checking whether the regular modules work properly is necessary and in addition a redundancy should also be given such that this monitoring module 120 checks whether the communication module 118 is working properly. This can be also done in a similar way as for the regular modules, i.e. the communication module 118 may send a heartbeat signal to the monitoring module 120. The monitoring module then checks whether this heartbeat signal is received with the expected repetition frequency. The repetition frequency of the monitoring module maybe 10 Hz or more, in particular 12 Hz.

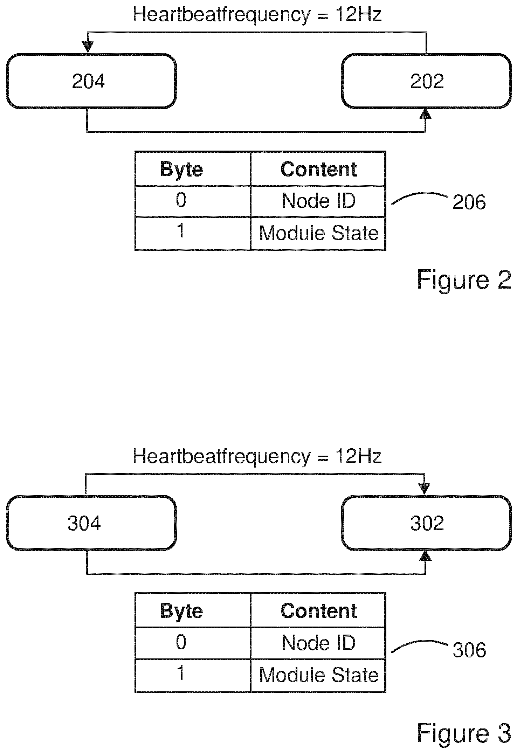

Figure 2 illustrates a control concept on how to handle the repetition frequency. This diagram illustrates a supervising node 202 and a CAN-node 204. The supervising node 202 represents the communication module 118, or a corresponding node of the communication module 118. The CAN-node 204 indicates that a CAN bus system is used and the CAN-node represents any regular module. I.e. could be any regular module of the regular modules explained with respect to Figure 1, and also other regular modules.

Figure 2 thus illustrates that the supervising node 202, thus the communication module 118 sends a repetition frequency, which can also be denominated as heartbeat frequency, as a set value to the CAN-node 204 and thus to one regular module. The value illustrated in Figure 2 is 12 Hz but it could also be another value and it could also be varying between the regular modules to which this value is sent.

Based on that repetition frequency received from the supervising node the CAN-node 204 sends with this repetition frequency a data frame to the supervising node 202. Such data frame is illustrated by the table 206 shown in Figure 2.

Accordingly, this data frame has a node ID and thus an identification number of the particular CAN-node, thus of the regular module, and has a module state. The module state could just be an okay or an error, or it could be an indication whether the particular regular module is in operation or in standby.

Accordingly, said data frame illustrated by table 206 is sent from the CAN-node 204, thus sent from a regular module to the supervising node 202, thus to the communication module, which could be the communication module 118 according to Figure 1. If this data frame is sent with the repetition frequency and also providing at least the status okay, the supervising node 202 identifies that there is no error in the particular regular module.

Figure 3 is similar to Figure 2 and shows also a supervising node 302 and a CAN-node 304. These can basically be the same as the supervising node 202 and the CAN-node 204 according to Figure 2. Basically, the only difference in this control concept shown in Fig-ure 3 is that the CAN-node 304 and thus the regular module which this CAN-node 304 represents, already has its repetition frequency and sends this repetition frequency to the supervising node 302. Accordingly, the supervising node 302 knows which repetition frequency to expect.

Based on that the concept works as shown in Figure 2, i.e. the CAN-node 304 sends a data frame with the repetition frequency and the data frame is illustrated by the table 306.

Accordingly, the supervising node 304 has no control of the heartbeat frequency. The node itself, i.e. the regular module reports in which frequency the heartbeat frame is sent. This has the advantage that there is a module specific handling and risks. For example, a temperature sent does not need to be supervised very fast because failures would not immediately result in critical situations. There is also a modular approach possible. That means independent development of each module with their own specification is possible and this enables an exchangeability of such modules. However, there could be an advantage that no central control of an over bus load is possible.

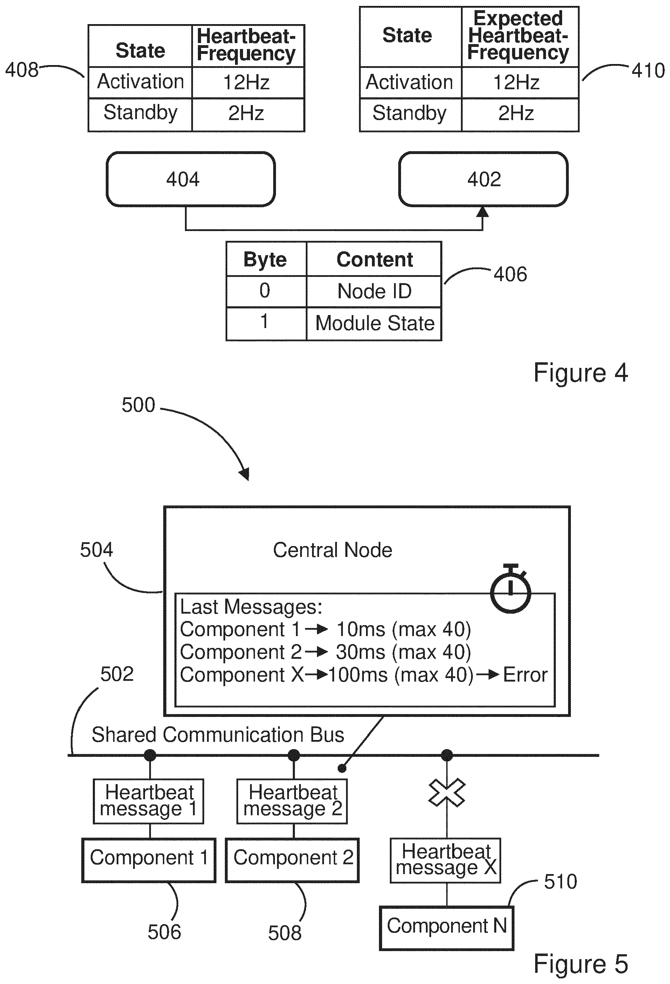

Another control concept of handling the repetition frequency is illustrated in Figure 4. Figure 4 also has a supervising node 402 and an CAN-node 404.

According to this concept, neither the supervising node sends a repetition frequency to the CAN-node nor sends the CAN-node its repetition frequency to the supervising node. Instead the repetition frequency depends on a state of the electro surgical generator, i.e. whether it is in activation or in standby. These different states have different repetition frequencies assigned to. In the given example there is 12 Hz for the activation and 2 Hz for the standby. This is illustrated in the first lookup table 408.

Based on that the CAN-node 404 sends a data frame with the repetition frequency to the supervising node 402. The general function of these two nodes and what they are representative for is explained in Figures 2 and 3. Accordingly, the CAN-node 404 is representative for any regular module and the supervising node 402 is representative for the communication module. The data frame sent over is also illustrated by the table 406 which has been explained with respect to table 206 in Figure 2.

However the used repetition frequency depends on the first lookup table 408 and accordingly said data frame represented by the table 406 is sent with the repetition frequency of 2 Hz if the generator is in standby and with the repetition frequency of 12 Hz if the generator is in an activation state.

The supervising node 402 is having a second lookup table 410. In this second lookup table 410 the expected heartbeat frequency and thus the expected repetition frequency is recorded. The expected heartbeat frequency according to the second lookup table 410 is thus corresponding to the given heartbeat frequency according to the first lookup table 408.

Therefore, the supervising node 402 checks if the CAN-node 404 sends the data frame according to table 406 with the correct repetition frequency, i.e. the correct heartbeat frequency according to its second lookup table 410.

To summarize Figure 4, the modules have a lookup table for heartbeat frequencies per module. There are two variants:
- The lookup table is fixed between all modules. Accordingly, It is a fixed and specified table which is the same on all nodes. All operating modes are globally the same. In particular the mode or status can be activated and standby.
- The lookup table is communicated for example during boot up with module specific parameters.

This table is the link between the heartbeat frequency and the operational mode of the module. The operational mode might be:
- Based on global system state for example generator is in activation mode or standby.
- Module dependent, e.g. instrument is currently not connected, so the repetition frequency, i.e. the heartbeat frequency can be reduced.

Advantages are as explained with respect to Figures 2 and 3. In addition, a very granular selection of surveillance time can be programmed. There is also a modular approach possible. I.e. there is possible an independent development of each module with their own specification enabling an exchangeability. There might be a small disadvantage such that the lookup table needs memory and management by surveillance node and it might be a more complex implementation with respect to the other possibilities explained above.

Figure 5 illustrates a check routine and load situation of the electro surgical generator using a communication bus. It shows a bus system 500 having a shared communication bus, i.e. a bus 502 to which a communication module 504 and three regular modules 506, 508 and 510 are connected. The communication and the illustrated checking whether any of the regular modules is without any malfunction is coordinated by the communication module 504. The communication module 504 can also be denominated as central node. This communication module frequently receives information signals from these three regular modules and of course from further modules as well, if any. In this illustration it is assumed that the communication module expects every regular module to respond or send data frames within an repetition time of 40 ms. Accordingly, it checks whether it receives messages from each regular module 506, 508 and 510 within this repetition time of 40 ms.

Based on that, there is a situation assumed that the first regular module 506 sends its message within 10 ms after its last message. Accordingly, this 10 ms is within the maximum repetition time of 40 ms.

The second regular module 508 sends its message within 30 ms after the last message and that is also within the limit of 40 ms for the repetition time.

However, the third regular module which may also be representative for any further regular modules, is sending its message after 100 ms. Accordingly, these 100 ms are bigger than the repetition time of 40 ms which is thus a maximum repetition time of 40 ms. Accordingly, for the third regular module 510 an error was identified and accordingly any error signal or safety actions will take place in the next step.

As a background of the invention it was also found that in a distributed system with multiple hardware and software components that communicate to each other, the overall system integrity must be ensured. If critical components fail, the system security or critical functions cannot be assured. Therefore, every component of a generator may incorporate mechanisms to supervise that every software and hardware component is working properly and reacts with a given output.

In principle a super-vising system receives a message that could be a heartbeat, hardware signal or digital message, from a component, i.e. a regular module, on a regular basis. If the message is not received in a given/specified time frame, that could be monitored using a timeout, the supervisor expects that the system is stalled and has to incorporate further actions to return to a stable system state. This can be a system reset or error handling.

In the current system the software components (called "tasks") are using this system. Every task sends a message to the system supervisor, which could be implemented in the communication module, and could be denominated as task supervisor. These messages could be sent at least every 60 ms. If a task is not sending a message in the given timeframe, the system is rebooted. An external Watchdog circuit supervises the task supervisor and the CPU. If the task supervisor does not reset the external watchdog, a hardware reset of the CPU could be triggered.

## Claims

1. Method for controlling an electro surgical generator (100) for controlling electro surgical instruments connected to the electro surgical generator, and the electro surgical generator comprises
- a plurality of interconnected regular modules, the plurality of interconnected regular modules comprising
- at least one socket module (104) for connecting the electro surgical instrument, and
- at least one first inverter module (112) for generating a feed signal for providing a high frequency energy for at least one electro surgical instrument connected to the socket module, wherein
- each regular module communicates at least with another regular module or with a communication module (118),
- each regular module is sending a communication frame at an individual repetition frequency and
- the individual repetition frequency of each regular module is different to the repetition frequencies of at least one, a plurality or all of the other regular modules.

2. Method according to claim 1, **characterized in that**
- the electro surgical generator further comprises the communication module for controlling a communication with the regular modules, and/or
- each regular module frequently sends a communication frame at its individual repetition frequency to the communication module and/or to another regular module.

3. Method according to claim 1 or 2, **characterized in that**
- the repetition frequency of at least one of the regular modules is given by the communication module, wherein
- the communication module sends an individual repetition frequency setting to the regular module and the regular module records the received repetition frequency setting, or
- the communication module controls the repetition frequency of a regular module by sending at the repetition frequency communication frames to the regular module, requesting an instantaneous answer and thereby forcing the regular module to answer at the repetition frequency.

4. Method according to any of the preceding claims **characterized in that**
at least one of the regular modules
- has its individual repetition frequency recorded in a memory as an individual repetition frequency setting, and
- provides its individual repetition frequency to the communication module and/or
- provides its individual repetition frequency to other regular modules whereby
- the regular modules or the communication module check whether the sent individual repetition frequency complies with a check-routine.

5. Method according to any of the preceding claims **characterized in that**
- the individual repetition frequency is used to check a reactivity of the corresponding regular module, wherein
- for checking the reactivity, a test signal is sent to the regular module to be tested, and it is monitored if the regular module answers the test signal at least at its repetition frequency.

6. Method according to any of the preceding claims **characterized in that**
- individual repetition frequencies of the regular modules are varied, in particular
- individual repetition frequencies are varied by the communication module, and/or
- individual repetition frequencies are varied depending on operational conditions of the electro surgical generator and/or the corresponding regular module.

7. Method according to any of the preceding claims **characterized in that**
- the individual repetition frequency is selected
- depending on the kind of regular module, in particular whether the regular module is involved in controlling energy of an electro surgical instrument connected to the electro surgical generator, and/or
- depending on an operating condition of the regular module, in particular whether the regular module is in operation or in standby.

8. Method according to any of the preceding claims **characterized in that**
- the plurality of interconnected regular modules comprises at least one further module of the list defined by
- an energy distribution module for controlling an energy supply from the at least one inverter module to the at least one socket module,
- a display module for displaying information for a user,
- an input module for receiving demands from the user, and
- at least a second inverter module for generating a feed signal for providing a high frequency energy for at least one electro surgical instrument with a different signal frequency than the first inverter module, and/or
- the electro surgical generator comprises a monitoring module for monitoring if the communication module operates properly.

9. Method according to any of the preceding claims **characterized in that**
- the regular modules and the communication module are interconnected using a bus system, in particular a serial and/or differential bus system.

10. Method according to any of the preceding claims **characterized in that**
- each regular module sends a frame comprising at least
- an identification code identifying the operational module, and
- an operation code, characterizing the operating state of the regular module, and optionally
- additional information including information of boot-up, error information, information on standby and/or information on a busy-status.

11. Method according to any of the preceding claims **characterized in that**
- an error-signal is generated if
- a regular module is in a mode of frequently sending a communication frame at its individual repetition frequency and
- the regular module is deviating from sending a communication frame at its individual repetition frequency by a predetermined minimum tolerance frequency, and/or if
- for checking a reactivity a test signal is sent to the regular module to be tested, and
- an answer of the regular module to the test signal is taking longer than a time defined by an inverse value of the repetition frequency of the tested regular module by a minimum time lag.

12. Electro surgical generator (100) for controlling electro surgical instruments connected to the electro surgical generation, comprising
- a plurality of interconnected regular modules, the plurality of interconnected regular modules comprising
- at least one socket module (104, 106) for connecting an electro surgical instrument, and
- at least one first inverter module (112) for generating a feed signal for providing a high frequency energy as a feed signal for at least one electro surgical instrument connected to the socket module, wherein
- each regular module is adapted for communicating at least with a communication module (118),
- each regular module is adapted to send a communication frame at an individual repetition frequency and
- the individual repetition frequency is varied depending on a state of the electro surgical generator, and in particular
- the electro surgical generator is adapted to execute a method according to any of the preceding claims.

13. Electro surgical generator according to claim 12, **characterized in that**
- the electro surgical generator further comprises the communication module for controlling a communication with the regular modules, and/or
- each regular module is adapted to frequently send a communication frame at its individual repetition frequency to the communication module and/or to another regular module.

14. Electro surgical generator according to claim 12 or 13, **characterized in that**
- the plurality of interconnected regular modules comprises at least one further module of the list defined by
- an energy distribution module for controlling an energy supply from the at least one inverter module to the at least one socket module,
- a display module for displaying information for a user,
- an input module for receiving demands from the user, and
- at least a second inverter module for generating a feed signal for providing a high frequency energy for at least one electro surgical instrument with a different signal frequency than the first inverter module and/or
- the electro surgical generator comprises a monitoring module for monitoring if the communication module operates properly.

15. Electro surgical generator according to any of claim 12 to 14, **characterized in that**
- the regular modules and the communication module are interconnected using a bus system, in particular a serial and/or differential bus system.

## Patentansprüche

1. Verfahren zur Steuerung eines elektrochirurgischen Generators (100) zur Steuerung elektrochirurgischer Instrumente, die mit dem elektrochirurgischen Generator verbunden sind, wobei der elektrochirurgische Generator Folgendes umfasst
- mehrere miteinander verbundene reguläre Module, wobei die mehreren miteinander verbundenen regulären Module Folgendes umfassen
- mindestens ein Steckermodul (104) zum Anschluss des elektrochirurgischen Instruments, und
- mindestens ein erstes Wechselrichtermodul (112) zur Erzeugung eines Speisesignals zur Bereitstellung von Hochfrequenzenergie für mindestens ein an das Steckermodul angeschlossenes elektrochirurgisches Instrument, wobei
- jedes reguläre Modul mindestens mit einem anderen regulären Modul oder mit einem Kommunikationsmodul (118) kommuniziert,
- jedes reguläre Modul einen Kommunikationsframe mit einer individuellen Wiederholungsfrequenz sendet und
- die individuelle Wiederholungsfrequenz jedes regulären Moduls unterscheidet sich von den Wiederholungsfrequenzen von mindestens einem, mehreren oder allen anderen regulären Modulen.

2. Verfahren nach Anspruch 1, **dadurch gekennzeichnet, dass**
- der elektrochirurgische Generator außerdem das Kommunikationsmodul zur Steuerung der Kommunikation mit den regulären Modulen umfasst, und/oder
- jedes reguläre Modul häufig einen Kommunikationsframe mit seiner individuellen Wiederholungsfrequenz an das Kommunikationsmodul und/oder an ein anderes reguläres Modul sendet.

3. Verfahren nach Anspruch 1 oder 2, **dadurch gekennzeichnet, dass**
- die Wiederholungsfrequenz mindestens eines der regulären Module durch das Kommunikationsmodul vorgegeben wird, wobei
- das Kommunikationsmodul eine individuelle Wiederholungsfrequenzeinstellung an das reguläre Modul sendet und das reguläre Modul die empfangene Wiederholungsfrequenzeinstellung aufzeichnet, oder
- das Kommunikationsmodul die Wiederholungsfrequenz eines regulären Moduls steuert, indem es an der Wiederholungsfrequenz Kommunikationsframes an das reguläre Modul sendet, eine sofortige Antwort anfordert und dadurch das reguläre Modul zwingt, mit der Wiederholungsfrequenz zu antworten.

4. Verfahren nach einem der vorstehenden Ansprüche, **dadurch gekennzeichnet, dass**
mindestens eines der regulären Module
- seine individuelle Wiederholungsfrequenz in einem Speicher als individuelle Wiederholungsfrequenzeinstellung gespeichert hat und
- seine individuelle Wiederholungsfrequenz dem Kommunikationsmodul zur Verfügung stellt und/oder
- seine individuelle Wiederholungsfrequenz an andere reguläre Module gibt, wobei
- die regulären Module oder das Kommunikationsmodul prüfen, ob die gesendete individuelle Wiederholungsfrequenz einer Prüfroutine entspricht.

5. Verfahren nach einem der vorstehenden Ansprüche, **dadurch gekennzeichnet, dass**
- die individuelle Wiederholungsfrequenz zur Überprüfung einer Reaktivität des entsprechenden regulären Moduls verwendet wird, wobei
- zur Überprüfung der Reaktivität ein Testsignal an das zu prüfende reguläre Modul gesendet wird, und überwacht wird, ob das reguläre Modul das Testsignal mindestens mit seiner Wiederholungsfrequenz beantwortet.

6. Verfahren nach einem der vorstehenden Ansprüche, **dadurch gekennzeichnet, dass**
- individuelle Wiederholungsfrequenzen der regulären Module variiert werden, insbesondere
- individuelle Wiederholungsfrequenzen durch das Kommunikationsmodul variiert werden, und/oder
- individuelle Wiederholungsfrequenzen je nach Betriebsbedingungen des elektrochirurgischen Generators und/oder des entsprechenden regulären Moduls variiert werden.

7. Verfahren nach einem der vorstehenden Ansprüche, **dadurch gekennzeichnet, dass**
- die individuelle Wiederholungsfrequenz gewählt wird
- abhängig von der Art des regulären Moduls, insbesondere ob das reguläre Modul an der Steuerung der Energie eines an den elektrochirurgischen Generator angeschlossenen elektrochirurgischen Instruments beteiligt ist, und/oder
- abhängig von einem Betriebszustand des regulären Moduls, insbesondere ob das reguläre Modul in Betrieb oder im Standby ist.

8. Verfahren nach einem der vorstehenden Ansprüche, **dadurch gekennzeichnet, dass**
- die mehreren miteinander verbundenen regulären Module mindestens ein weiteres Modul aus der Liste aufweisen, umfassend,
- ein Energieverteilungsmodul zur Steuerung der Energieversorgung von dem mindestens einen Wechselrichtermodul zu dem mindestens einen Steckermodul,
- ein Anzeigemodul zur Darstellung von Informationen für einen Benutzer,
- ein Eingabemodul zur Entgegennahme von Anforderungen durch den Benutzer und
- mindestens ein zweites Wechselrichtermodul zur Erzeugung eines Speisesignals zur Bereitstellung von Hochfrequenzenergie für mindestens ein elektrochirurgisches Instrument mit einer anderen Signalfrequenz als das erste Wechselrichtermodul, und/oder
- der elektrochirurgische Generator ein Überwachungsmodulumfasst, mit dem überwacht werden kann, ob das Kommunikationsmodul ordnungsgemäß funktioniert.

9. Verfahren nach einem der vorstehenden Ansprüche, **dadurch gekennzeichnet, dass**
- die regulären Module und das Kommunikationsmodul über ein Bussystem, insbesondere ein serielles und/oder differentielles Bussystem, miteinander verbunden sind.

10. Verfahren nach einem der vorstehenden Ansprüche, **dadurch gekennzeichnet, dass**
- jedes reguläre Modul einen Frame sendet, mindestens umfassend
- einen Identifikationscode, der das Betriebsmodul identifiziert, und
- einen Betriebscode, der den Betriebszustand des regulären Moduls charakterisiert, und optional
- Zusatzinformationen, einschließlich Informationen über das Hochfahren, Fehlerinformationen, Informationen über den Bereitschaftszustand und/oder Informationen über den Besetzt-Zustand.

11. Verfahren nach einem der vorstehenden Ansprüche, **dadurch gekennzeichnet, dass**
- ein Fehlersignal erzeugt wird, wenn
- ein reguläres Modul sich in einem Modus befindet, in dem es häufig einen Kommunikationsframe mit seiner individuellen Wiederholungsfrequenz sendet und
- das reguläre Modul vom Senden eines Kommunikationsframes mit seiner individuellen Wiederholungsfrequenz um eine vorgegebene Mindesttoleranzfrequenz abweicht, und/oder wenn
- zur Überprüfung der Reaktivität ein Testsignal an das zu prüfende reguläre Modul gesendet wird, und
- eine Antwort des regulären Moduls auf das Testsignal länger dauert als eine Zeit, die durch einen Kehrwert der Wiederholungsfrequenz des geprüften regulären Moduls um eine Mindestzeitspanne definiert ist.

12. Elektrochirurgischer Generator (100) zur Steuerung elektrochirurgischer Instrumente, die an den elektrochirurgischen Generator angeschlossen sind, umfassend
- mehrere miteinander verbundene reguläre Module, wobei die mehreren miteinander verbundenen regulären Module Folgendes umfassen:
- mindestens ein Steckermodul (104, 106) zum Anschluss eines elektrochirurgischen Instruments und
- mindestens ein erstes Wechselrichtermodul (112) zur Erzeugung eines Speisesignals zur Bereitstellung einer Hochfrequenzenergie als Speisesignal für mindestens ein an das Steckermodul angeschlossenes elektrochirurgisches Instrument, wobei
- jedes reguläre Modul dazu vorbereitet ist, dass es zumindest mit einem Kommunikationsmodul (118) kommunizieren kann,
- jedes reguläre Modul dazu vorbereitet ist, einen Kommunikationsframe mit einer individuellen Wiederholungsfrequenz zu senden und
- die individuelle Wiederholungsfrequenz in Abhängigkeit von einem Zustand des elektrochirurgischen Generators variiert wird, und zwar insbesondere
- der elektrochirurgische Generator zur Durchführung eines Verfahrens nach einem der vorstehenden Ansprüche geeignet ist.

13. Elektrochirurgischer Generator nach Anspruch 12, **dadurch gekennzeichnet, dass**
- der elektrochirurgische Generator außerdem das Kommunikationsmodul zur Steuerung der Kommunikation mit den regulären Modulen umfasst, und/oder
- jedes reguläre Modul in der Lage ist, häufig einen Kommunikationsframe mit seiner individuellen Wiederholungsfrequenz an das Kommunikationsmodul und/oder an ein anderes reguläres Modul zu senden.

14. Elektrochirurgischer Generator nach Anspruch 12 oder 13, **dadurch gekennzeichnet, dass**
- die mehreren miteinander verbundenen regulären Module mindestens ein weiteres Modul umfassen aus der Liste, die definiert ist durch
- ein Energieverteilungsmodul zur Steuerung der Energieversorgung von dem mindestens einen Wechselrichtermodul zu dem mindestens einen Steckermodul,
- ein Anzeigemodul zur Darstellung von Informationen für einen Benutzer,
- ein Eingabemodul zur Entgegennahme von Anforderungen durch den Benutzer und
- mindestens ein zweites Wechselrichtermodul zur Erzeugung eines Speisesignals zur Bereitstellung einer Hochfrequenzenergie für mindestens ein elektrochirurgisches Instrument mit einer anderen Signalfrequenz als das erste Wechselrichtermodul und/oder
- der elektrochirurgische Generator ein Überwachungsmodulumfasst, mit dem überwacht werden kann, ob das Kommunikationsmodul ordnungsgemäß funktioniert.

15. Elektrochirurgischer Generator nach einem der Ansprüche 12 bis 14, **dadurch gekennzeichnet, dass**
- die regulären Module und das Kommunikationsmodul über ein Bussystem, insbesondere ein serielles und/oder differentielles Bussystem, miteinander verbunden sind.

## Revendications

1. Procédé de commande d'un générateur électrochirurgical (100) pour commander des instruments électrochirurgicaux connectés au générateur électrochirurgical, et le générateur électrochirurgical comporte
- une pluralité de modules réguliers interconnectés, la pluralité de modules réguliers interconnectés comportant
- au moins un module de prise (104) pour connecter l'instrument électrochirurgical, et
- au moins un premier module d'onduleur (112) pour générer un signal d'alimentation pour fournir une énergie haute fréquence pour au moins un instrument électrochirurgical connecté au module de prise, dans lequel
- chaque module régulier communique au moins avec un autre module régulier ou avec un module de communication (118),
- chaque module régulier envoie une trame de communication à une fréquence de répétition individuelle et
- la fréquence de répétition individuelle de chaque module régulier est différente des fréquences de répétition d'au moins un, d'une pluralité ou de tous les autres modules réguliers.

2. Procédé selon la revendication 1, **caractérisé en ce que**
- le générateur électrochirurgical comporte en outre le module de communication pour commander une communication avec les modules réguliers, et/ou
- chaque module régulier envoie fréquemment une trame de communication à sa fréquence de répétition individuelle au module de communication et/ou à un autre module régulier.

3. Procédé selon la revendication 1 ou 2, **caractérisé en ce que**
- la fréquence de répétition d'au moins un des modules réguliers est donnée par le module de communication, dans lequel
- le module de communication envoie un réglage de fréquence de répétition individuelle au module régulier et le module régulier enregistre le réglage de fréquence de répétition reçu, ou
- le module de communication commande la fréquence de répétition d'un module régulier en envoyant à la fréquence de répétition des trames de communication au module régulier, en demandant une réponse instantanée et en forçant ainsi le module régulier à répondre à la fréquence de répétition.

4. Procédé selon l'une quelconque des revendications précédentes **caractérisé en ce qu'**au moins un des modules réguliers
- a sa fréquence de répétition individuelle enregistrée dans une mémoire en tant que réglage de fréquence de répétition individuelle, et
- fournit sa fréquence de répétition individuelle au module de communication et/ou
- fournit sa fréquence de répétition individuelle à d'autres modules réguliers où
- les modules réguliers ou le module de communication vérifient si la fréquence de répétition individuelle envoyée est conforme à une routine de vérification.

5. Procédé selon l'une quelconque des revendications précédentes **caractérisé en ce que**
- la fréquence de répétition individuelle est utilisée pour vérifier une réactivité du module régulier correspondant, dans lequel
- pour vérifier la réactivité, un signal de test est envoyé au module régulier à tester, et on surveille si le module régulier répond au signal de test au moins à sa fréquence de répétition.

6. Procédé selon l'une quelconque des revendications précédentes **caractérisé en ce que**
- les fréquences de répétition individuelles des modules réguliers subissent une variation, en particulier
- des fréquences de répétition individuelles subissent une variation au moyen du module de communication, et/ou
- des fréquences de répétition individuelles subissent une variation en fonction des conditions de fonctionnement du générateur électrochirurgical et/ou du module régulier correspondant.

7. Procédé selon l'une quelconque des revendications précédentes **caractérisé en ce que**
- la fréquence de répétition individuelle est sélectionnée
- en fonction du type de module régulier, en particulier si le module régulier est impliqué dans la commande de l'énergie d'un instrument électrochirurgical connecté au générateur électrochirurgical, et/ou
- en fonction d'une condition de fonctionnement du module régulier, en particulier si le module régulier est en fonctionnement ou en veille.

8. Procédé selon l'une quelconque des revendications précédentes **caractérisé en ce que**
- la pluralité de modules réguliers interconnectés comporte au moins un autre module de la liste défini par
- un module de distribution d'énergie pour commander une alimentation en énergie de l'au moins un module d'onduleur vers l'au moins un module de prise,
- un module d'affichage d'informations pour un utilisateur,
- un module d'entrée pour recevoir des demandes de l'utilisateur, et
- au moins un deuxième module d'onduleur pour générer un signal d'alimentation pour fournir une énergie haute fréquence pour au moins un instrument électrochirurgical avec une fréquence de signal différente du premier module d'onduleur, et/ou
- le générateur électrochirurgical comporte un module de surveillance pour surveiller si le module de communication fonctionne correctement.

9. Procédé selon l'une quelconque des revendications précédentes **caractérisé en ce que**
- les modules réguliers et le module de communication sont interconnectés à l'aide d'un système de bus, en particulier un système de bus série et/ou différentiel.

10. Procédé selon l'une quelconque des revendications précédentes **caractérisé en ce que**
- chaque module régulier envoie une trame comportant au moins
- un code d'identification identifiant le module opérationnel, et
- un code de fonctionnement, caractérisant l'état de fonctionnement du module régulier, et facultativement
- des informations supplémentaires incluant des informations de démarrage, des informations d'erreur, des informations sur la veille et/ou des informations sur un état occupé.

11. Procédé selon l'une quelconque des revendications précédentes **caractérisé en ce que**
- un signal d'erreur est généré si
- un module régulier est dans un mode où il envoie fréquemment une trame de communication à sa fréquence de répétition individuelle et
- le module régulier s'écarte de l'envoi d'une trame de communication à sa fréquence de répétition individuelle d'une fréquence de tolérance minimale prédéterminée, et/ou si
- pour vérifier une réactivité, un signal de test est envoyé au module régulier à tester, et
- une réponse du module régulier au signal de test dure plus longtemps qu'un temps défini par une valeur inverse de la fréquence de répétition du module régulier testé d'un décalage temporel minimal.

12. Générateur électrochirurgical (100) pour commander des instruments électrochirurgicaux connectés à la génération électrochirurgicale, comportant
- une pluralité de modules réguliers interconnectés, la pluralité de modules réguliers interconnectés comportant
- au moins un module de prise (104, 106) pour connecter un instrument électrochirurgical, et
- au moins un premier module d'onduleur (112) pour générer un signal d'alimentation pour fournir une énergie haute fréquence en tant que signal d'alimentation pour au moins un instrument électrochirurgical connecté au module de prise, dans lequel
- chaque module régulier est adapté pour communiquer au moins avec un module de communication (118),
- chaque module régulier est adapté pour envoyer une trame de communication à une fréquence de répétition individuelle et
- la fréquence de répétition individuelle subit une variation en fonction d'un état du générateur électrochirurgical, et en particulier
- le générateur électrochirurgical est adapté pour exécuter un procédé selon l'une quelconque des revendications précédentes.

13. Générateur électrochirurgical selon la revendication 12, **caractérisé en ce que**
- le générateur électrochirurgical comporte en outre le module de communication pour commander une communication avec les modules réguliers, et/ou
- chaque module régulier est adapté pour envoyer fréquemment une trame de communication à sa fréquence de répétition individuelle au module de communication et/ou à un autre module régulier.

14. Générateur électrochirurgical selon la revendication 12 ou 13, **caractérisé en ce que**
- la pluralité de modules réguliers interconnectés comporte au moins un autre module de la liste défini par
- un module de distribution d'énergie pour commander une alimentation en énergie de l'au moins un module d'onduleur vers l'au moins un module de prise,
- un module d'affichage d'informations pour un utilisateur,
- un module d'entrée pour recevoir des demandes de l'utilisateur, et
- au moins un deuxième module d'onduleur pour générer un signal d'alimentation pour fournir une énergie haute fréquence pour au moins un instrument électrochirurgical avec une fréquence de signal différente du premier module d'onduleur et/ou
- le générateur électrochirurgical comporte un module de surveillance pour surveiller si le module de communication fonctionne correctement.

15. Générateur électrochirurgical selon l'une quelconque des revendications 12 à 14, **caractérisé en ce que**
- les modules réguliers et le module de communication sont interconnectés à l'aide d'un système de bus, en particulier un système de bus série et/ou différentiel.
